# EUROPÄISCHE PATENTSCHRIFT

(11) **EP 1 197 507 B1**
(45) Veröffentlichungstag und Bekanntmachung des Hinweises auf die Patenterteilung: **01.06.2005**
(21) Anmeldenummer: 01122609.9
(22) Anmeldetag: 27.09.2001
(51) Int. Cl.: C08G 18/38, C07C 227/08

(54) **Stabilisierte Mono- und Polyasparaginsäureester**
Stabilised Mono- and Polyaspartic acid ester
Ester d'acide mono- ou polyaspartique stabilisé

(30) Priorität: 11.10.2000 DE 10050137
(43) Veröffentlichungstag der Anmeldung: 17.04.2002
(73) Patentinhaber: Bayer MaterialScience AG, 51368 Leverkusen (DE)
(72) Erfinder: Gertzmann, Rolf, Dr., 51377 Leverkusen (DE); Schmalstieg, Lutz, Dr., 50676 Köln (DE); Groth, Stefan, Dr., 51375 Leverkusen (DE)

(56) Entgegenhaltungen:
- EP-A- 0 667 362
- EP-A- 0 816 326

## Beschreibung

Die vorliegende Erfindung betrifft Zusammensetzungen auf Basis von Mono- und Polyasparaginsäureester, ein Verfahren zu deren Herstellung und ihr Einsatz als reaktive Komponente für Polyisocyanate in Zweikomponenten-Polyurethan-Systemen.

Zweikomponenten(2K)-Beschichtungsmittel, die als Bindemittel eine Polyisocyanatkomponente in Kombination mit einer gegenüber Isocyanatgruppen reaktionsfähigen Reaktivkomponente, insbesondere einer Polyhydroxylkomponente enthalten, sind seit langem bekannt. Sie eignen sich zur Herstellung von hochwertigen Überzügen, die hart, elastisch, abrieb- und lösemittelbeständig und vor allem auch witterungsstabil eingestellt werden können.

Innerhalb dieser 2K-Polyurethan-Beschichtungstechnologie haben sich in letzter Zeit bestimmte, estergruppenhaltige, sekundäre Polyamine etabliert, die sich in Kombination mit Lackpolyisocyanaten insbesondere als Bindemittel in lösemittelarmen oder -freien highsolid-Beschichtungsmitteln eignen und eine rasche Aushärtung der Beschichtungen bei niedrigen Temperaturen ermöglichen.

Bei diesen sekundären Polyaminen handelt es sich um die sogenannten Polyasparaginsäureester, wie sie beispielhaft in der EP-A 0 403 921 beschrieben werden. Ihre Verwendung allein oder im Gemisch mit weiteren, gegenüber Isocyanatgruppen reaktionsfähigen Komponenten in 2K-PUR-Beschichtungsmitteln wird beispielsweise in der EP-A 0 403 921, EP-A 0 639 628, EP-A 0 667 362, EP-A 0 689 881, US-A 5 214 086, EP-A 0 699 696, EP-A 0 596 360, EP-A 0 893 458, DE-A 19 701 835 und US-A 5 243 012 beschrieben.

Die Synthese der Polyasparaginsäureester erfolgt über eine Addition von primären Aminen an eine aktivierte Kohlenstoff-Doppelbindung vinyloger Carbonylverbindungen, wie beispielsweise in Malein-oder Fumarsäureestern enthaltend, die in der Literatur hinreichend beschrieben ist (Houben Weyl, Meth. d. Org. Chemie Bd. 11/1, 272 (1957), Usp. Khim. 1969, 38, 1933).

Es hat sich gezeigt, dass diese Reaktion während der Synthese (z.B. nach 24 Stunden unter Rühren bei 60°C) nicht vollständig abläuft. Der Umsatz der Reaktion hängt entscheidend von dem Typ des primären Polyamins ab. Der Umsetzungsgrad (gemessen anhand der Konzentration an freiem, nicht umgesetzten Malein- und Fumarsäureester) nach 1 Tag mit 1,5-Diamino-2-methyl-pentan liegt bei 90 bis 93 %. Im Gegensatz dazu liegt der Umsetzungsgrad bei Verwendung eines cycloaliphatischen Polyamins mit sterisch gehinderten Aminogruppen (z.B. 4,4'-Diamino-3,3'-dimethyldicyclohexylmethan) nur bei 77 %. Die vollständige oder nahezu vollständige Umsetzung wird erst nach einigen Monaten erreicht.

Diese unvollständig umgesetzten Produkte enthalten nicht umgesetzte primäre Aminogruppen und gegebenenfalls auch freie primäre Polyamine sowie den entsprechenden Anteil an nicht umgesetztem Malein- oder Fumarsäureester. Dies hat zur Folge, dass die Produkte nach der Herstellung bei anschließender Lagerung weiter reagieren und damit die Viskosität der Reaktionsmischung stetig bis zum Erreichen des vollständigen Umsatz ansteigt. Zudem verringert sich mit Abnahme der Konzentration primärer Aminogruppen die Reaktivität des Produkts gegenüber Isocyanaten. Somit ist die Einstellung einer reproduzierbaren Topfzeit nicht gewährleistet.

Im Stand der Technik sind bereits verschiedene Lösungsansätze des Problems beschrieben worden, welche jedoch noch nicht zu endgültig zufriedenstellenden Ergebnissen führen.

Zunächst besteht grundsätzlich die Möglichkeit die Reaktionszeit zu verlängern oder die Reaktionstemperatur zu erhöhen. Ersteres scheidet aus ökonomischen Gründen aus. Eine Erhöung der Reaktionstemperatur auf z.B. 80°C oder sogar 100°C führt wiederum zu einer drastischen Erhöhung der Farbzahl des Produktes.

In der EP-A 0 667 362 und US-A 5 243 012 wird eine Verlängerung der Topfzeit von 2K-PUR-Bindemitteln auf Basis von Polyisocyanaten und Polyasparaginsäureestern durch Zusätze von Zeolithen bzw. Organozinn(IV)-Verbindungen beschrieben. Diese Maßnahmen haben jedoch nur behelfsmäßigen Charakter und beeinträchtigen andere Eigenschaften, beispielsweise kann eine Trübung des Lacks oder eine Beschleunigung der NCO/OH-Reaktion im Bindemittel auftreten.

In der US-A 5 821 326 wird beschrieben, dass mit fünfgliedrigen aromatischen Ringverbindungen die Reaktion zur Herstellung der Mono- und Polyasparaginsäureester katalysierbar ist. Die dort eingesetzten Katalysatoren erlauben zwar eine schnellere Umsetzung der Komponenten, doch ist in keinem angeführten Beispiel ein vollständiger Umsatz des Reaktionsgemisches innerhalb einer Reaktionszeit von vier Tagen bei 60°C erreicht worden. Bei der anschließenden Lagerung des Produktgemisches kommt es dann aufgrund der unvollständigen Reaktion zu einem Viskositätsanstieg des Produkts.

In der US-A 5 126 170 wird der überschüssige Fumarsäureester mittels Destillation entfernt und somit der Reaktion entzogen. Dieses Verfahren ist allerdings zeit- und energieaufwendig und bietet daher keine Basis für einen technischen Prozess.

Es besteht somit nach wie vor das Bedürfnis nach Mono- und Polyasparaginsäureester-Systeme, die eine verbesserte Viskositäts- und damit Lagerstabilität aufweisen, ohne die Qualität der resultierenden Bindemittel bzw. Beschichtungen negativ zu beeinträchtigen.

Es wurde nun gefunden, dass die Zugabe einer Thiolverbindung zu einer unvollständig umgesetzten Reaktionsmischung im Laufe der Lagerung zu einem deutlich geringerem Viskositätsanstieg des Produkts führt und das Endprodukt keine Geruchsbelästigung durch die eingesetzten Thiolverbindungen aufweist. Die Zugabe der Thiolverbindung bewirkt, dass der nicht umgesetzte Teil an vinyloger Carbonylverbindung einer weiteren Reaktion mit dem primären Aminen nicht mehr zur Verfügung steht.

Gegenstand der Erfindung ist somit eine Zusammensetzungen enthaltend Mono- und Polyasparaginsäureester der allgemeinen Formel (I), 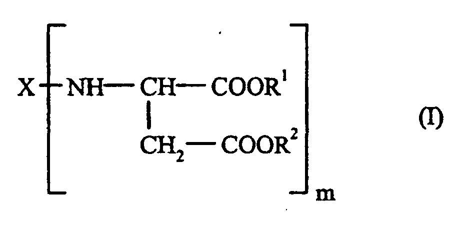 in welcher
- X: für einen m-wertigen, gegebenenfalls ein oder mehrere Heteroatome enthaltenden organischen Rest steht, wie er durch Entfernung der primären Aminogruppe bzw. -gruppen aus einem entsprechenden, (cyclo)aliphatisch bzw. araliphatisch gebundene primäre Aminogruppen aufweisenden Mono- oder Polyamin des Molekulargewichtsbereichs 60 bis 6000 erhalten werden kann, und der weitere, gegenüber Isocyanatgruppen reaktive und/oder bei Temperaturen bis 100°C inerte funktionelle Gruppen enthalten kann,
- R¹ und R²: für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen und
- m: für eine ganze Zahl ≥ 1, bevorzugt ≥ 2 und besonders bevorzugt = 2 steht,
sowie ein Additionsprodukt bestehend aus einer Verbindung der allgemeinen Formel (II) (Komponente A),

R¹OOC-CH=CH-COOR² (II)

wobei die Reste R¹ und R² die oben genannte Bedeutung haben,
und einer Thiolverbindung der allgemeinen Formel (III) (Komponente B),

[HS]ₙ-R³ (III),

in welcher
- R³: einen n-valenten, gegebenenfalls ein oder mehrere Heteroatome aufweisenden organischen Rest darstellt, der andere funktionelle Gruppen enthalten kann, die gegenüber Isocyanaten reaktiv oder inert sind und
- n: für eine ganze Zahl ≥ 1 und ≤ 4 steht.

Die erfindungsgemäßen Zusammensetzungen sind Produkte mit verbesserter Viskositätsstabilität.

Weiterer Gegenstand der Erfindung ist ein Verfahren zur Herstellung von Mono- und Polyasparaginsäureester der allgemeinen Formel (I), 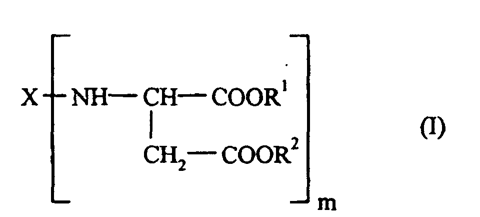 in welcher
- X: für einen m-wertigen, gegebenenfalls ein oder mehrere Heteroatome enthaltenden organischen Rest steht, wie er durch Entfernung der primären Aminogruppe bzw. -gruppen aus einem entsprechenden, (cyclo)aliphatisch bzw. araliphatisch gebundene primäre Aminogruppen aufweisenden Mono - oder Polyamin des Molekulargewichtsbereichs 60 bis 6000 erhalten werden kann, und der weitere, gegenüber Isocyanatgruppen reaktive und/oder bei Temperaturen bis 100°C inerte funktionelle Gruppen enthalten kann,
- R¹ und R²: für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen und
- m: für eine ganze Zahl ≥ 1, bevorzugt ≥ 2 und besonders bevorzugt = 2 steht,
durch Umsetzung von Mono- oder Polyaminen der allgemeinen Formel (IV) (Komponente C),

X[-NH₂]ₘ (IV)

wobei X und m die oben genannte Bedeutung haben,
mit Verbindungen der allgemeinen Formel (II) (Komponente A),

R¹OOC-CH=CH-COOR² (II)

wobei die Reste R¹ und R² die oben genannte Bedeutung haben,
dadurch gekennzeichnet, dass mindestens eine Thiolverbindung der allgemeinen Formel (III) (Komponente B),

[HS]ₙ-R³ (III),

in welcher
- R³: einen n-valenten, gegebenenfalls ein oder mehrere Heteroatome aufweisenden organischen Rest darstellt, der andere funktionelle Gruppen enthalten kann, die gegenüber Isocyanaten reaktiv oder inert sind und
- n: für eine ganze Zahl ≥ 1 und ≤ 4 steht,
bei einem Restgehalt an Doppelbindungen von 2 bis 15 %, gemessen an den zu Beginn der Reaktion eingesetzten Doppelbindungen, zur Reaktion zugesetzt wird.

Vorzugsweise erfolgt das erfindungsgemäße Verfahren zur Herstellung Mono- bzw. Polyasparaginsäureester der allgemeinen Formel (I) in zwei Schritten. Im ersten Schritt werden die Komponenten A und C bei Temperaturen zwischen 0°C und 100°C, vorzugsweise 20 bis 80°C und besonders bevorzugt 20 bis 60°C, in einem Verhältnis der Äquivalente der primären Aminogruppen der Komponente C zu den C=C-Doppelbindungsäquivalenten der Komponente A von 1:1,2 bis 1,2:1, vorzugsweise jedoch 1:1,05 bis 1,05:1 bis zu einem Restgehalt an Doppelbindungen, gemessen an den zu Reaktionsbeginn vorhandenen Doppelbindungen von 2 bis 15 %, bevorzugt 5 bis 10 % umgesetzt. Im zweiten Schritt werden in einem Temperaturbereich von 0°C bis 100°C, vorzugsweise 20°C bis 80°C und besonders bevorzugt 20°C bis 60° C nicht umgesetzte Doppelbindungsäquivalente der Komponente A mit den Thiolgruppen der Komponente B im molaren Verhältnis von 1,5:1 bis 1:1 vorzugsweise von 1,2:1 bis 1:1 und besonders bevorzugt von 1,05:1 umgesetzt.

Eine weitere geeignete Ausführungsform des erfindungsgemäßen Verfahrens besteht in der Herstellung der Mono bzw. Polyasparaginsäureester in drei Schritten, wobei zwei verschiedene Amine der allgemeinen Formel (IV) als Komponente C1 und C2 eingesetzt werden. Im ersten Schritt werden die Komponenten A und C1 bei Temperaturen zwischen 0°C und 100°C, vorzugsweise 20 bis 80°C und besonders bevorzugt 20 bis 60°C, in einem Verhältnis der Äquivalente der primären Aminogruppen der Komponente C1 zu den C=C-Doppelbindungsäquivalenten der Komponente A von 1:1,3 bis 1:2 vorzugsweise jedoch 1:1,5 bis 1:1,7 bis zu einem Restgehalt an primären Aminogruppen von 0 bis 15 %, bevorzugt 0 bis 10 % umgesetzt. Anschließend wird die Mischung in einem zweiten Schritt mit einer Komponente C2 in einem Temperaturbereich von 0°C bis 100°C, vorzugsweise 20°C bis 80°C und besonders bevorzugt 20°C bis 60°C umgesetzt, so dass das Verhältnis der Summe der Äquivalente der Aminogruppen aus Komponente C1 und C2 zu den Doppelbindungsäquivalenten der Komponente A 1:1,2 bis 1,2:1, vorzugsweise jedoch 1:1,05 bis 1,05:1 beträgt. Die Reaktion wird soweit getrieben, dass noch 2 bis 15 %, bevorzugt 5 bis 10 % der Doppelbindungen übrig sind. Im dritten Schritt werden in einem Temperaturbereich von 0°C bis 100°C, vorzugsweise 20°C bis 80°C und besonders bevorzugt 20°C bis 60°C nicht umgesetzte Doppelbindungsäquivalente der Komponente A mit den Thiolgruppen der Komponente B im molaren Verhältnis von 1,5:1 bis 1:1, vorzugsweise von 1,2:1 bis 1:1 und besonders bevorzugt von 1,05:1 umgesetzt.

Im erfindungsgemäßen Verfahren als Komponente C einsetzbar sind im Prinzip alle bekannten Mono- und Polyamine mit mindestens einer primären Aminogruppe, die der allgemeinen Formel (IV) entsprechen.

Als primäre Monoamine gemäß Formel (IV) (m=1) sind beispielsweise diejenigen Monoamine gut geeignet, die in ihrem organischen Rest X eine oder mehrere, weitere funktionelle Gruppen enthalten, die entweder gegenüber Isocyanatgruppen reaktiv oder inert sind. Beispielhaft genannt seien in diesem Zusammenhang aminofunktionelle Silane wie beispielsweise 3-Aminopropyltrimethoxysilan, 3-Aminopropyltriethoxysilan oder auch Aminoalkohole wie z. B. Ethanolamin, Propanolamin oder Isopropanolamin.

Im erfindungsgemäßen Verfahren als Komponente C bevorzugt einsetzbar sind Polyamine der allgemeinen Formel (IV) worin m für eine Zahl größer oder gleich 2 steht. Beispiele für bevorzugt einsetzbare Verbindungen sind Ethylendiamin, 1,2-Diaminopropan, 1,4-Diaminobutan, 2,5-Diamino-2,5-dimethylhexan, 1,5-Diamino-2-methylpentan (Dytek® A, Fa DuPont), 1,6-Diaminohexan, 2,2,4- und/oder 2,4,4-Trimethyl-1,6-diaminohexan, 1,11-Diaminoundecan, 1,12-Diaminododecan oder Triaminononan.

Ebenfalls einsetzbar sind höhermolekulare Polyetherpolyamine mit aliphatisch gebundenen, primären Aminogruppen, wie sie beispielsweise unter der Bezeichnung Jeffamin® von der Firma Huntsman vertrieben werden.

Besonders bevorzugt werden im erfindungsgemäßen Verfahren Polyamine der allgemeinen Formel (IV) eingesetzt, worin m = 2 bedeutet und X für einen cyclischen Kohlenwasserstoffrest mit mindestens einem cyclischen Kohlenstoffring steht. Beispiele für besonders bevorzugt einsetzbare Diamine sind 1-Amino-3,3,5-trimethyl-5-aminomethylcyclohexan (IPDA), 2,4- und/oder 2,6-Hexahydrotoluylendiamin (H₆TDA), Isopropyl-2,4-diaminocyclohexan und/oder Isopropyl-2,6-diaminocyclohexan, 1,3-Bis-(aminomethyl)-cyclohexan, 2,4'- und/oder 4,4'-Diamino-dicyclohexylmethan, 3,3'-Dimethyl-4,4'-diamino-dicyclohexylmethan (Laromin® C 260, Fa. BASF AG), die Isomeren, eine Methylgruppe als Kernsubstituenten aufweisenden Diaminodicyclohexylmethane (= C-Monomethyl-diaminodicyclohexylmethane), 3(4)-Aminomethyl-1-methylcyclohexylamin (AMCA) sowie araliphatische Diamine, wie z.B. 1,3-Bis-(aminomethyl)-benzol.

Als Komponente A werden im erfindungsgemäßen Verfahren Malein- oder Fumarsäureester der allgemeinen Formel (II) eingesetzt, worin R¹ und R² für gleiche oder verschiedene organische Reste mit je 1 bis 18 Kohlenstoffatomen stehen. Bevorzugt stehen R¹ und R² unabhängig voneinander für lineare oder verzweigte Alkylreste mit 1 bis 8 Kohlenstoffatomen.

Beispiele für als Komponente A geeignete Verbindungen sind Maleinsäuredimethylester, -diethylester, -di-n- oder -isopropylester, -di-n-butylester, -di-2-ethylhexylester oder die entsprechenden Fumarsäureester.

Als Komponente B werden im erfindungsgemäßen Verfahren Mercaptoverbindungen der allgemeinen Strukturformel (III) eingesetzt. Bevorzugte Mercaptoverbindungen umfassen beispielsweise Trimethylolpropan-tri-(3-mercaptopropionat), Pentaerythrit-tetra-(3-mercaptopropionat), Glykol-di-(3-mercaptopropionat), Glykol-dimercaptoacetat, Trimethylolpropan-trithioglykolat, 2-Ethylhexylthioglycolat, n- und/oder Isopropylthioglycolat, n-, iso- und/oder tert-Butylthioglycolat, Mercaptodiethylether, Cyclohexylmercaptan, Ethandithiol, 1,4-Butandithiol, 1,6-Hexandithiol, Dodecandithiol, Didodecandithiol, Dimercaptobenzothiazol, Allylmercaptan, Benzylmercaptan, 2-Mercaptoethanol, 2,3-Dimercaptopropanol, α,α'-Dimercapto-p-xylol, Thiosalicylsäure, Thiomilchsäure, Mercaptopropionsäure, Mercaptoessigsäure, ein Mercaptopyridin, Dithioerythrit, 6-Ethoxy-2-mercaptobenzothiazol, d-Limonen-dimercaptan sowie flüssige Polysulfide wie sie von der Fa. Marton Int. GmbH unter der Bezeichnung LP vertrieben werden oder Gemische aus diesen.

Besonders bevorzugt einsetzbar als Komponente B sind Ester der Thioglycolsäure mit linearen oder verzweigten Alkoholen mit mindestens 4 Kohlenstoffatomen. Ebenfalls geeignet sind Thiole, die weitere, gegenüber Isocyanaten reaktive Gruppen enthalten. Beispielhaft seien hier 2,3-Dimercapto-1-propanol, 2-Mercaptoethanol, 3-Mercapto-1,2-propandiol, Ethylenglycolmonothioglycolat, 2-Hydroxyethyl-3-mercaptopropionat, 6-Mercapto-1-hexanol, Glycerinmonothioglycolat, 4-Mercaptobutanol, 11-Mercapto-1-undecanol, Cysteinol und Glyeryl-3-mercaptopropionat genannt. Außerdem sind aliphatische monofunktionelle Thiole mit Molgewichten über 146 g/Mol, wie z.B. 1-Octanthiol, Dodecanthiol und Didodecanthiol geeignet. Weniger bevorzugt sind Mischungen der genannten Thiolverbindungen.

Die erfindungsgemäße Darstellung der Mono- oder Polyasparaginsäureester der Formel (I) kann sowohl in Lösung als auch lösemittelfrei durchgeführt werden. Der Zusatz von Lösemittel kann jedoch auch erst nach Beendigung des Syntheseprozesses erfolgen, z.B. um die Viskosität zu senken. Als Lösemittel kommen im Prinzip alle organischen Lösemittel in Frage, bevorzugt natürlich die in der Beschichtungstechnologie verwendeten Lösemittel. Beispielhaft, aber nicht ausschließlich gemeint, seien Aceton, Methylethylketon, Methylisobutylketon, n-Butylacetat, Methoxypropylacetat, Toluol, Xylol sowie höhere aromatische Lösemittel, wie sie von der Fa. Exxon-Chemie unter der Bezeichnung Solvesso® vertrieben werden, genannt.

In einer bevorzugten Ausführungsform der Erfindung enthält die erfindungsgemäße Zusammensetzung
a) 98 bis 55 Gew. % eines Mono- und Polyasparaginsäureesters der allgemeinen Formel (I),
b) 45 bis 1 Gew. % eines Additionsproduktes bestehend aus einer Komponente A der allgemeinen Formel (II) und einer Komponente B der allgemeinen Formel (III),
c) 0 bis 3 Gew. % freier Komponente A der allgemeinen Formel (II),
d) 0 bis 1 Gew. % freie Komponente C der allgemeinen Formel (IV),
mit der Maßgabe, dass die Summe von a) bis d) 100 Gew.-% ergeben,
sowie gegebenenfalls freie Komponente B der allgemeinen Formel (III) und gegebenenfalls die üblichen Hilfs-und Zusatzstoffe.

In einer besonders bevorzugten Ausführungsform der Erfindung enthält die erfindungsgemäße Zusammensetzung
a) 97 bis 71 Gew.-% eines Mono- und Polyasparaginsäureesters der allgemeinen Formel (I),
b) 29 bis 3 Gew.-% eines Additionsproduktes bestehend aus einer Komponente A der allgemeinen Formel (II) und einer Komponente B der allgemeinen Formel (III),
c) 0 bis 0,5 Gew.-% freier Komponente A der allgemeinen Formel (II),
d) 0 bis 0,5 Gew.-% freie Komponente C der allgemeinen Formel (IV),
mit der Maßgabe, dass die Summe von a) bis d) 100 Gew.-% ergeben,
sowie gegebenenfalls freie Komponente B der allgemeinen Formel (III) und gegebenenfalls die üblichen Hilfs-und Zusatzstoffe.

Die erfindungsgemäßen Zusammensetzungen sind direkt nach Abschluß des Syntheseprozesses einsatzfähig und im Hinblick auf Reaktivität über einen Zeitraum mehrerer Monate stabil und zeigen eine deutlich verbesserte Viskositätsstabilität. Aufgrund ihrer niedrigeren Eigenviskosität im Vergleich zu den Mono- und Polyasparaginsäureestern die nach Verfahren des Standes der Technik hergestellt wurden, stellen die erfindungsgemäßen Zusammensetzungen wertvolle Reaktionspartner für Polyisocyanate in lösemittelarmen oder -freien Zweikomponenten-Polyurethan-Lacken und Beschichtungen dar.

Gegenstand der Erfindung ist somit auch die Verwendung der erfindungsgemäßen Zusammensetzungen als Reaktivkomponente in Zweikomponenten-Polyurethan-Systemen oder zur Herstellung von Prepolymeren.

Die Zweikomponenten(2K)- Polyurethan-Systeme, enthaltend die erfindungsgemäßen Zusammensetzungen, können dann als Beschichtungsmitteln zur Herstellung von Überzügen verwendet werden.

### Beispiele

Alle Prozentangaben beziehen sich, sofern nicht anders beschrieben, auf das Gewicht.

### A) Analytik der Polyasparaginsäureester

Der Umsatz lässt sich anhand der Signale der Methylenprotonen des Fumarsäureester (in den sich Maleinsäureester basenkatalytisch umlagert) und des Asparaginsäureesters mittles ¹H - NMR -Spektroskopie (400 MHz) bestimmen. Zur Aufnahme des Spektrums wurde die Probe in CDCl₃ gelöst.

Die Viskositäten wurden an einem Viskosimeter der Fa. Physika (Kegel Platte) bestimmt. Das Schergefälle betrug 40 -s⁻¹.

### B) Darstellung der Polyasparaginsäureester

### I) Allgemeine Vorschrift zur Darstellung der Polyasparaginsäureester (Vergleichsbeispiele)

In einem 1 l-Vierhalskolben mit Rührer, Tropftrichter, Rückflusskühler und Innenthermometer werden 1 mol (2 Val) des Diamins (Komponente C) unter Stickstoffatmosphäre vorgelegt und unter Rühren werden 2 Mol Maleinsäurediethylester (344,36 g, Komponente A) so zugesetzt, dass 50°C in der Reaktionsmischung erreicht, aber nicht wesentlich überschritten werden. Anschließend läßt man 60 bis 180 h bei 60°C nachrühren. Während der gesamten Reaktionszeit wird ein schwacher Stickstoffstrom über das Reaktionsgemisch geleitet.

### I.1) Beispiel 1 (Vergleichsbeispiel):

Vorgehensweise wie unter (I):

| | |
|---|---|
| 210,4 g | 4,4'-Diaminodicyclohexylmethan (PACM 20; 1 mol) |
| 344,4 g | Maleinsäurediethylester (2 mol) |

| | |
|---|---|
| Reaktionstemperatur | 60°C |
| Reaktionsdauer | 90 h |
| Umsatz | 95 % |
| Viskosität [mPa s/23°C] | 1070 |

### Lagerung: 4 Wochen

Nach vierwöchiger Lagerung bei 50°C weist das Produkt einen **Umsatz von 97 %** und eine **Viskosität von 1400 mPa s/23°C** auf.

### I.2) Beispiel 2 (Vergleichsbeispiel):

| | |
|---|---|
| 238,4 g | 3,3'-Dimethyl-4,4'-diamino-dicyclohexylmethan (Laromin C 260® , BASF; 1 Mol) |
| 344,4 g | Maleinsäurediethylester (MSDEE; 2 Mol) |

| | |
|---|---|
| Reaktionstemperatur | 60 °C |
| Reaktionsdauer | 90 h |
| Umsatz | 90 % |
| Viskosität [mPa s/23 °C] | 870 |

### Lagerung: 3 Wochen

Nach dreiwöchiger Lagerung bei 50°C weist das Produkt einen **Umsatz von 95 %** und eine **Viskosität von 1630 mPa s/23°C** auf.

### II) Allgemeine Vorschrift zur Darstellung der Polyasparaginsäureester unter Zugabe von Thioglycolverbindungen (erfindungsgemäßes Beispiel)

Vorgehensweise wie unter I) beschrieben. Im Anschluß wird das Produkt auf Raumtemperatur gekühlt und der verbleibende Fumarsäurediethylester zu 90 mol-% mit der Thiolverbindung versetzt, so dass 60°C nicht überschritten werden. Die Mischung wird für 4 h bei Raumtemperatur vermischt und abgefüllt.

### II.1) Beispiel 3 (erfindungsgemäßes Beispiel):

In den nachstehend beschriebenen, erfindungsgemäßen Beispielen 3, 4, 5 und 6 wird nach 90 h Reaktionszeit die Thiolverbindung zugesetzt.

| | |
|---|---|
| 210,4 g | 4,4'-Diaminodicyclohexylmethan (PACM 20; 1 mol) |
| 344,4 g | Maleinsäurediethylester (2 mol) |
| 36,7 g | 2-Ethylhexylthioglykolat |

| | |
|---|---|
| Reaktionstemperatur | 60°C |
| Reaktionsdauer | 94 h |
| Umsatz | 99 % |
| Viskosität [mPa s/23°C] | 1000 |

### Lagerung:

**3 Monate bei Raumtemperatur:** Umsatz = 99 %; Viskosität: 1200 mPa s/23°C
**4 Wochen bei 50°C**: Umsatz = 99 %; Viskosität: 1200 mPa s/23°C

### II.2) Beispiel 4 (erfindungsgemäßes Beispiel):

| | |
|---|---|
| 210,4 g | 4,4'-Diaminodicyclohexylmethan (PACM 20; 1 mol) |
| 344,4 g | Maleinsäurediethylester (2 mol) |
| 36,4 g | 1-Dodecanthiol |

| | |
|---|---|
| Reaktionstemperatur | 60°C |
| Reaktionsdauer | 94 h |
| Umsatz | 99 % |
| Viskosität [mPa s/23°C] | 1000 |

### Lagerung:

**3 Monate bei Raumtemperatur:** Umsatz = 99 %; Viskosität: 1000 mPa s/23°C
**4 Wochen bei 50°C**: Umsatz = 99 %;Viskosität: 1080 mPa s/23°C

### II.3) Beispiel 5 (erfindungsgemäßes Beispiel):

| | |
|---|---|
| 238,4 g | 3,3'-Dimethyl-4,4'-diamino-dicyclohexylmethan (Laromin C 260® ; 1 mol) |
| 344,4 g | Maleinsäurediethylester (MSDEE; 2 Mol) |
| 73,4 g | 2-Ethylhexylthioglykolat |

| | |
|---|---|
| Reaktionstemperatur | 60°C |
| Reaktionsdauer | 94 h |
| Umsatz | 98 % |
| Viskosität [mPa s/23°C] | 900 |

### Lagerung:

**3 Wochen bei 50 °C:** Umsatz = 99 %; Viskosität: 1000 mPa s/23°C

### II.4) Beispiel 6 (erfindungsgemäßes Beispiel):

| | |
|---|---|
| 238,4 g | 3,3'-Dimethyl-4,4'-diamino-dicyclohexylmethan (Laromin C 260® ; 1 mol) |
| 344,4 g | Maleinsäurediethylester (MSDEE; 2 mol) |
| 72,8 g | 1-Dodecanthiol |

| | |
|---|---|
| Reaktionstemperatur | 60 °C |
| Reaktionsdauer | 94 h |
| Umsatz | 98 % |
| Viskosität [mPa s/23 °C] | 900 |

### Lagerung:

**3 Monate bei Raumtemperatur:** Umsatz = 99 %; Viskosität: 980 mPa s/23 °C

### C) Gelzeiten von Beschichtungszusammensetzungen

In den folgenden Beispielen werden Beschichtungen durch Kombination der in den Beispielen 1 bis 5 beschriebenen Polyasparaginsäureestern und Polyisocyanaten hergestellt. Zur Herstellung der Beschichtungen und zur Bestimmung der Gelzeiten werden die Polyasparaginsäureester mit einem aus 1,6-Hexamethylendiisocyanat hergestellten Polyisocyanat (Desmodur® N 3600 der Bayer AG; Viskosität: 1200mPas/23°C, NCO-Gehalt: 23,4 %, Äquivalentgewicht: 180 g) in einem NCO:NH Verhältnis von 1,05:1 umgesetzt. Die Gelzeit ist das Intervall vom Vermischung der beiden Komponenten bis zum Zeitpunkt an dem die Mischung nicht mehr gerührt werden kann.

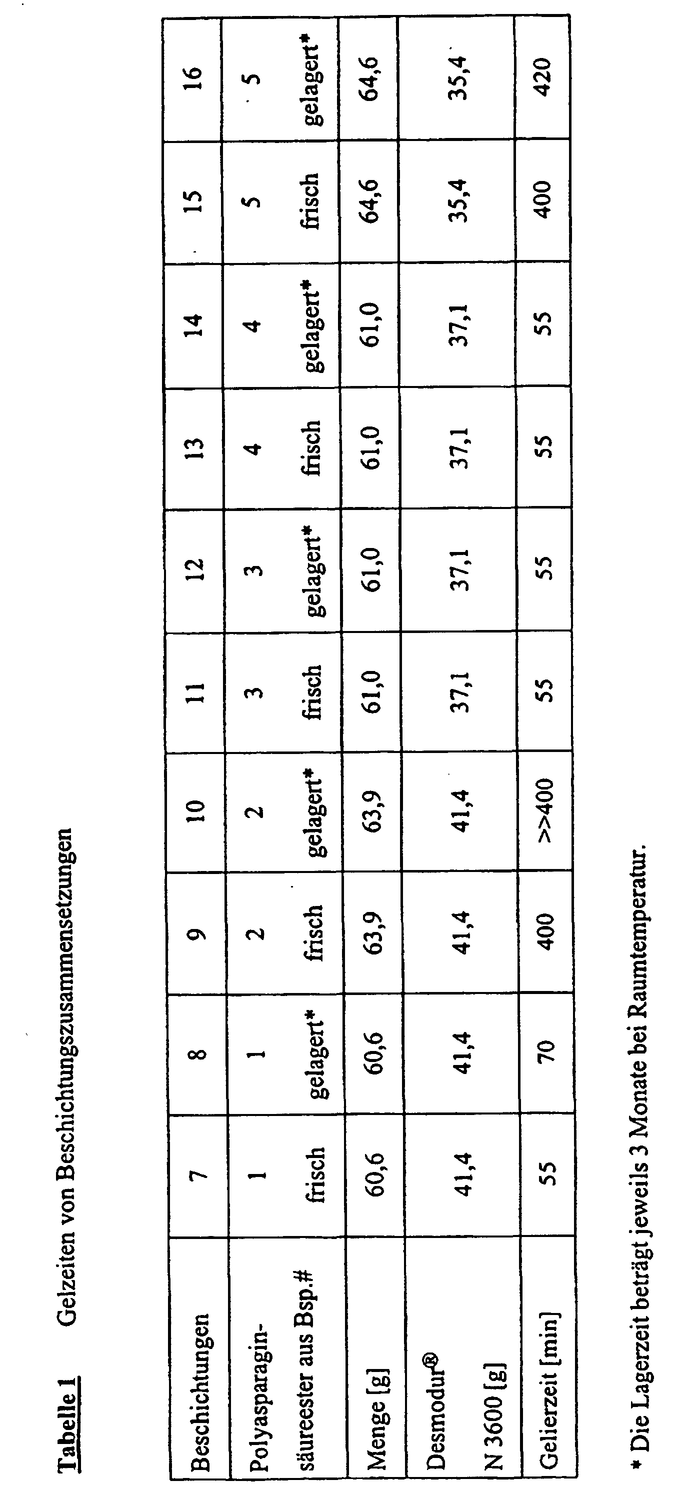

Durch den Vergleich der in Tab. 1 angegebenen Gelierzeiten der Mischung zur Herstellung der Beschichtungen 7,8 bzw. 9 und 10 (Vergleichsbeispiele) mit denen der Mischungen zur Herstellung der Beschichtungen 11-14 bzw. 15 und 16 (erfindugsgemäße Asparaginsäureester) läßt sich die höhere Produktstabilität der Asparaginsäureester erkennen.

### D) Messung der Shore-Härten

Die Messungen erfolgten nach DIN 53505.

| | | | | | | | | | | |
|---|---|---|---|---|---|---|---|---|---|---|
| Beschichtungen | 7 | 8 | 9 | 10 | 11 | 12 | 13 | 14 | 15 | 16 |
| Shore Härte | 80 | 81 | 60 | 65 | 80 | 80 | 80 | 81 | 61 | 63 |
| | Polyasparaginsäureester aus Vergleichsbeispielen | | | | Polyasparaginsäureester aus erfindungsgemäßen Beispielen | | | | | |

## Patentansprüche

1. Zusammensetzungen enthaltend Mono- und Polyasparaginsäureester der allgemeinen Formel (I), 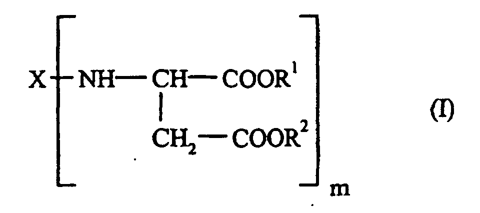 in welcher
X für einen m-wertigen, gegebenenfalls ein oder mehrere Heteroatome enthaltenden organischen Rest steht, wie er durch Entfernung der primären Aminogruppe bzw. -gruppen aus einem entsprechenden, (cyclo)aliphatisch bzw. araliphatisch gebundene primäre Aminogruppen aufweisenden Mono- oder Polyamin des Molekulargewichtsbereichs 60 bis 6000 erhalten werden kann, und der weitere, gegenüber Isocyanatgruppen reaktive und/oder bei Temperaturen bis 100°C inerte funktionelle Gruppen enthalten kann,
R¹ und R² für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen und
m für eine ganze Zahl ≥ 1 steht,
sowie ein Additionsprodukt bestehend aus einer Verbindung der allgemeinen Formel (II) (Komponente A),
R¹OOC-CH=CH-COOR² (II)
wobei die Reste R¹ und R² die oben genannte Bedeutung haben,
und einer Thiolverbindung der allgemeinen Formel (III) (Komponente B),
[HS]ₙ-R³ (III),
in welcher
R³ einen n-valenten, gegebenenfalls ein oder mehrere Heteroatome aufweisenden organischen Rest darstellt, der andere funktionelle Gruppen enthalten kann, die gegenüber Isocyanaten reaktiv oder inert sind und
n für eine ganze Zahl ≥ 1 und ≤ 4 steht.

2. Verfahren zur Herstellung von Mono- und Polyasparaginsäureester der allgemeinen Formel (I), 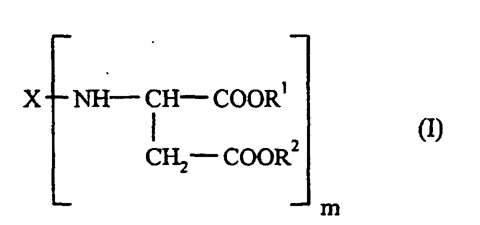 in welcher
X für einen m-wertigen, gegebenenfalls ein oder mehrere Heteroatome enthaltenden organischen Rest steht, wie er durch Entfernung der primären Aminogruppe bzw. -gruppen aus einem entsprechenden, (cyclo)aliphatisch bzw. araliphatisch gebundene primäre Aminogruppen aufweisenden Mono- oder Polyamin des Molekulargewichtsbereichs 60 bis 6000 erhalten werden kann, und der weitere, gegenüber Isocyanatgruppen reaktive und/oder bei Temperaturen bis 100°C inerte funktionelle Gruppen enthalten kann,
R¹ und R² für gleiche oder verschiedene organische Reste, vorzugsweise für gleiche oder verschiedene Alkylreste mit je 1 bis 18 Kohlenstoffatomen und ganz besonders bevorzugt für gleiche oder verschiedene Alkylreste mit je 1 bis 8 Kohlenstoffatomen stehen und
m für eine ganze Zahl ≥ 1 steht,
durch Umsetzung von Mono- oder Polyaminen der allgemeinen Formel (IV) (Komponente C),
X[-NH₂]ₘ (IV)
wobei X und m die oben genannte Bedeutung haben,
mit Verbindungen der allgemeinen Formel (II) (Komponente A),
R¹OOC-CH=CH-COOR² (II)
wobei die Reste R¹ und R² die oben genannte Bedeutung haben,
**dadurch gekennzeichnet, dass** mindestens eine Thiolverbindung der allgemeinen Formel (III) (Komponente B),
[HS]ₙ-R³ (III),
in welcher
R³ einen n-valenten, gegebenenfalls ein oder mehrere Heteroatome aufweisenden organischen Rest darstellt, der andere funktionelle Gruppen enthalten kann, die gegenüber Isocyanaten reaktiv oder inert sind und
n für eine ganze Zahl ≥ 1 und ≤ 4 steht,
bei einem Restgehalt an Doppelbindungen von 2 bis 15 %, gemessen an den zu Beginn der Reaktion eingesetzten Doppelbindungen, zur Reaktion zugesetzt wird.

3. Verfahren nach Anspruch 2, **dadurch gekennzeichnet, dass** mindestens eine Thiolverbindung der allgemeinen Formel (III) (Komponente B),
[HS]ₙ-R³ (III),
in welcher
R³ einen n-valenten, gegebenenfalls ein oder mehrere Heteroatome aufweisenden organischen Rest darstellt, der andere funktionelle Gruppen enthalten kann, die gegenüber Isocyanaten reaktiv oder inert sind und
n für eine ganze Zahl ≥ 1 und ≤ 4 steht,
bei einem Restgehalt an Doppelbindungen von 5 bis 10 %, gemessen an den zu Beginn der Reaktion eingesetzten Doppelbindungen, zur Reaktion zugesetzt wird.

4. Verfahren nach Anspruch 2, **dadurch gekennzeichnet, dass** in einem ersten Schritt die Komponenten A und C in einem Verhältnis der Äquivalente der primären Aminogruppen der Komponente C zu den C=C-Doppelbindungsäquivalenten der Komponente A von 1:1,2 bis 1,2:1 umgesetzt werden und in einem zweiten Schritt nicht umgesetzte Doppelbindungsäquivalente der Komponente A mit den Thiolgruppen der Komponente B im molaren Verhältnis von 1,5:1 bis 1:1umgesetzt werden.

5. Zusammensetzungen nach Anspruch 1 enthaltend
a) 98 bis 55 Gew.-% eines Mono- und Polyasparaginsäureesters der allgemeinen Formel (I),
b) 45 bis 1 Gew.-% eines Additionsproduktes bestehend aus einer Komponente A der allgemeinen Formel (II) und einer Komponente B der allgemeinen Formel (III),
c) 0 bis 3 Gew.-% freier Komponente A der allgemeinen Formel (II),
d) 0 bis 1 Gew.-% freie Komponente C der allgemeinen Formel (IV),
mit der Maßgabe, dass die Summe von a) bis d) 100 Gew.-% ergeben,
sowie gegebenenfalls freie Komponente B der allgemeinen Formel (III) und gegebenenfalls die üblichen Hilfs-und Zusatzstoffe.

6. Verwendung von Zusammensetzungen nach Anspruch 1 als Reaktivkomponente in Zweikomponenten-Polyurethan-Systemen.

7. Verwendung von Zusammensetzungen nach Anspruch 1 zur Herstellung von Prepolymeren.

8. Zweikomponenten-Polyurethan-Systeme enthaltend Zusammensetzungen nach Anspruch 1.

## Claims

1. Compositions comprising monoaspartic acid esters and polyaspartic acid esters of the general formula (I) 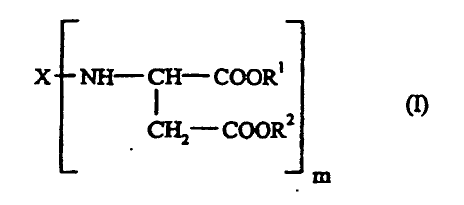 in which
X represents an m-valent organic radical, optionally containing one or more heteroatoms, as may be obtained by removing the primary amino group or groups from a corresponding monoamine or polyamine in the molecular weight range from 60 to 6000 containing (cyclo)aliphatically and/or araliphatically bound primary amino groups, and which may contain further functional groups that are reactive with respect to isocyanate groups and/or are inert at temperatures up to 100°C,
R¹ and R² represent identical or different organic radicals, preferably identical or different alkyl radicals each having 1 to 18 carbon atoms, and very preferably identical or different alkyl radicals each having 1 to 8 carbon atoms, and
m represents an integer ≥ 1,
and also an addition product composed of a compound of the general formula (II) (component A),
R¹OOC-CH=CH-COOR² (II)
where the radicals R¹ and R² are as defined above,
and a thiol compound of the general formula (III) (component B),
[HS]ₙ-R³ (III)
in which
R³ represents an n-valent organic radical optionally having one or more heteroatoms, which radical may contain other functional groups that are reactive or inert with respect to isocyanates, and
n represents an integer ≥ 1 and ≤ 4.

2. Process for preparing monoaspartic acid esters and polyaspartic acid esters of the general formula (I) 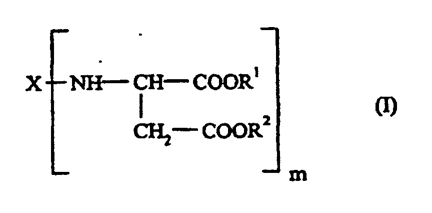 in which
X represents an m-valent organic radical, optionally containing one or more heteroatoms, as may be obtained by removing the primary amino group or groups from a corresponding monoamine or polyamine in the molecular weight range from 60 to 6000 containing (cyclo)aliphatically and/or araliphatically bound primary amino groups, and which may contain further functional groups that are reactive with respect to isocyanate groups and/or are inert at temperatures up to 100°C,
R¹ and R² represent identical or different organic radicals, preferably identical or different alkyl radicals each having 1 to 18 carbon atoms, and very preferably identical or different alkyl radicals each having 1 to 8 carbon atoms, and
m represents an integer ≥ 1,
by reacting monoamines or polyamines of the general formula (IV) (component C),
X[-NH₂]ₘ (IV)
where X and m are as defined above,
with compounds of the general formula (II) (component A),
R¹OOC-CH=CH-COOR² (II)
where the radicals R¹ and R² are as defined above,
**characterized in that** at least one thiol compound of the general formula (III) (component B),
[HS]ₙ-R³ (III)
in which
R³ represents an n-valent organic radical optionally having one or more heteroatoms, which radical may contain other functional groups that are reactive or inert with respect to isocyanates, and
n represents an integer ≥ 1 and ≤ 4,
is added to the reaction at a residual content of double bonds of 2 to 15%, measured in terms of the double bonds used at the start of the reaction.

3. Process according to Claim 2, **characterized in that** at least one thiol compound of the general formula (III) (component B),
[HS]ₙ-R³ (III)
in which
R³ represents an n-valent organic radical optionally having one or more heteroatoms, which radical may contain other functional groups that are reactive or inert with respect to isocyanates, and
n represents an integer ≥ 1 and ≤ 4,
is added to the reaction at a residual content of double bonds of 5 to 10%, measured in terms of the double bonds used at the start of the reaction.

4. Process according to Claim 2, **characterized in that** in a first stage the components A and C are reacted in a ratio of the equivalents of the primary amino groups of the component C to the C=C double bond equivalents of the component A of 1:1.2 to 1.2:1 and in a second stage unreacted double bond equivalents of the component A are reacted with the thiol groups of the component B in a molar ratio of 1.5:1 to 1:1.

5. Compositions according to Claim 1 comprising
a) 98 to 55% by weight of a mono- and polyaspartic acid ester of the general formula (I),
b) 45 to 1% by weight of an addition product composed of a component A of the general formula (II) and of a component B of the general formula (III),
c) 0 to 3% by weight of free component A of the general formula (II),
d) 0 to 1% by weight of free component C of the general formula (IV),
with the proviso that the sum of a) to d) makes 100% by weight,
and also, optionally, free component B of the general formula (III) and optionally the customary auxiliaries and additives.

6. Use of compositions according to Claim 1 as a reactive component in two-component polyurethane systems.

7. Use of compositions according to Claim 1 for preparing prepolymers.

8. Two-component polyurethane systems comprising compositions according to Claim 1.

## Revendications

1. Compositions contenant des esters d'acide mono- ou polyaspartique de la formule générale (I), 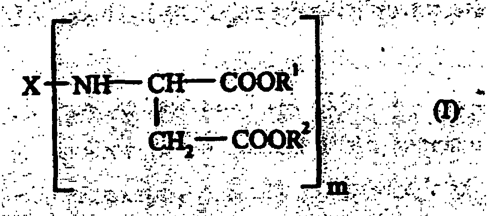 dans laquelle
X représente un radical organique m-valent, contenant éventuellement un ou plusieurs hétéroatomes, tel que celui qui peut être obtenu en éliminant le groupe ou les groupes amino primaire(s) d'une mono- ou d'une polyamine correspondante, présentant des groupes amino primaires à liaison (cyclo)aliphatique ou araliphatique, du domaine de poids moléculaire compris entre 60 et 6 000, et qui peut renfermer d'autres groupes fonctionnels réactifs vis-à-vis des groupes isocyanate et/ou inertes à l'égard de ceux-ci aux températures allant jusqu'à 100 °C,
R¹ et R² correspondent à des radicaux organiques identiques ou différents, de préférence des radicaux alkyle identiques ou différents comportant chacun 1 à 18 atomes de carbone et avec une préférence toute particulière des radicaux alkyle identiques ou différents comportant chacun 1 à 8 atomes de carbone et
m équivaut à un nombre entier ≥ 1,
ainsi qu'un produit d'addition constitué d'un composé de la formule générale (II) (composant A),
R¹OOC-CH=CH-COOR² (II)
dans laquelle les radicaux R¹ et R² possèdent la signification mentionnée ci-dessus,
et d'un composé thiolique de la formule générale (III) (composant B),
[HS]ₙ-R³ (III),
dans laquelle
R³ représente un radical organique n-valent présentant éventuellement un ou plusieurs hétéroatomes, qui peut contenir d'autres groupes fonctionnels qui sont réactifs ou inertes vis-à-vis des isocyanates et
n correspond à un nombre entier ≥ 1 et ≤ 4.

2. Procédé de préparation d'esters d'acide mono- ou polyaspartique de la formule générale (I), 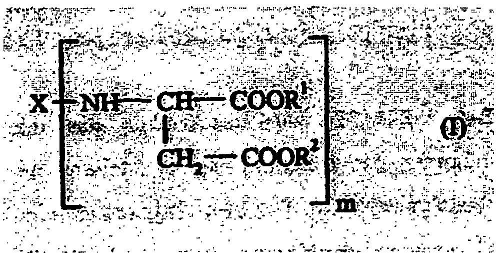 dans laquelle
X représente un radical organique m-valent, contenant éventuellement un ou plusieurs hétéroatomes, tel que celui qui peut être obtenu en éliminant le groupe ou les groupes amino primaire(s) d'une mono- ou d'une polyamine correspondante présentant des groupes amino primaires à liaison (cyclo)aliphatique ou araliphatique, du domaine de poids moléculaire compris entre 60 et 6 000 et qui peut renfermer d'autres groupes fonctionnels réactifs vis-à-vis des groupes isocyanate et/ou inertes à l'égard de ceux-ci aux températures allant jusqu'à 100 °C,
R¹ et R² correspondent à des radicaux organiques identiques ou différents, de préférence des radicaux alkyle identiques ou différents comportant chacun 1 à 18 atomes de carbone et avec une préférence toute particulière des radicaux alkyle identiques ou différents comportant chacun 1 à 8 atomes de carbone et
m équivaut à un nombre entier ≥ 1,
en faisant réagir des mono- ou des polyamines de la formule générale (IV) (composant C),
X[-NH₂]ₘ (IV)
dans laquelle X et m possèdent la signification mentionnée ci-dessus,
avec des composés de la formule générale (II) (composant A),
R¹OOC-CH=CH-COOR² (II)
dans laquelle les radicaux R¹ et R² possèdent la signification mentionnée ci-dessus,
**caractérisé en ce que** l'on ajoute à la réaction au moins un composé thiolique de la formule générale (III) (composant B),
[HS]ₙ-R³ (III),
dans laquelle
R³ représente un radical organique n-valent présentant éventuellement un ou plusieurs hétéroatomes, qui peut contenir d'autres groupes fonctionnels qui sont réactifs ou inertes vis-à-vis des isocyanates et
n correspond à un nombre entier ≥ 1 et ≤ 4,
avec une teneur résiduelle en doubles liaisons de 2 à 15 %, déterminée d'après les doubles liaisons mises en oeuvre au début de la réaction.

3. Procédé selon la revendication 2, **caractérisé en ce que** l'on ajoute à la réaction au moins un composé thiolique de la formule générale (III) (composant B),
[HS]ₙ-R³ (III),
dans laquelle
R³ représente un radical organique n-valent présentant éventuellement un ou plusieurs hétéroatomes, qui peut contenir d'autres groupes fonctionnels qui sont réactifs ou inertes vis-à-vis des isocyanates et
n correspond à un nombre entier ≥ 1 et ≤ 4.
avec une teneur résiduelle en doubles liaisons de 5 à 10 %, déterminée d'après les doubles liaisons mises en oeuvre au début de la réaction.

4. Procédé selon la revendication 2, **caractérisé en ce que** dans une première étape, les composants A et C sont mis à réagir dans un rapport des équivalents des groupes amino primaires du composant C par rapport aux équivalents des doubles liaisons C=C du composant A de 1:1,2 à 1,2:1 et dans une deuxième étape, les équivalents des doubles liaisons du composant A non entrés en réaction sont mis à réagir avec les groupes thiol du composant B, dans un rapport molaire de 1,5:1 à 1:1.

5. Compositions selon la revendication 1, contenant
a) 98 à 55 % en poids d'un ester d'acide mono- ou polyaspartique de la formule générale (I),
b) 45 à 1 % en poids d'un produit d'addition constitué d'un composant A de la formule générale (II) et d'un composant B de la formule générale (III),
c) 0 à 3 % en poids d'un composant A libre de la formule générale (II),
d) 0 à 1 % en poids d'un composant C libre de la formule générale (IV),
à condition que la somme de a) à d) fasse 100 %,
ainsi que, le cas échéant, le composant B libre de la formule générale (III) et, le cas échéant, les adjuvants et les additifs habituels.

6. Utilisation des compositions selon la revendication 1 comme composant réactif dans des systèmes à base de polyuréthane à deux composants.

7. Utilisation des compositions selon la revendication 1 pour préparer des prépolymères.

8. Systèmes à base de polyuréthane à deux composants contenant des compositions selon la revendication 1.
